(19) Europäisches Patentamt
European Patent Office
Office européen des brevets

(11) Veröffentlichungsnummer : **0 142 799**
**B1**

(12) **EUROPÄISCHE PATENTSCHRIFT**

(45) Veröffentlichungstag der Patentschrift : **27.07.88**

(21) Anmeldenummer : **84113559.3**

(22) Anmeldetag : **09.11.84**

(51) Int. Cl.[4] : **C 07 D265/30**, C 07 D211/14, C 07 D279/12, C 07 D211/74, C 07 D223/04, C 07 D295/02, A 01 N 43/84

# (54) Ammoniumsalze von Polysäuren, Verfahren zu ihrer Herstellung, Fungizide, die diese Salze enthalten und Verfahren zur Bekämpfung von Pilzen mit diesen Salzen.

(30) Priorität : **19.11.83 DE 3341829**

(43) Veröffentlichungstag der Anmeldung : **29.05.85 Patentblatt 85/22**

(45) Bekanntmachung des Hinweises auf die Patenterteilung : **27.07.88 Patentblatt 88/30**

(84) Benannte Vertragsstaaten : **AT BE CH DE FR GB IT LI NL SE**

(56) Entgegenhaltungen :
**DE-A- 2 461 513**
**DE-A- 2 700 680**
**DE-B- 1 173 722**

(73) Patentinhaber : **BASF Aktiengesellschaft**
**Carl-Bosch-Strasse 38**
**D-6700 Ludwigshafen (DE)**

(72) Erfinder : **Ammermann, Eberhard, Dr.**
**Sachsenstrasse 3**
**D-6700 Ludwigshafen (DE)**
Erfinder : **Buschmann, Ernst, Dr.**
**Georg-Ludwig-Krebs-Strasse 10**
**D-6700 Ludwigshafen (DE)**
Erfinder : **Ley, Gregor, Dr.**
**In den Trankstuecken**
**D-6719 Wattenheim (DE)**
Erfinder : **Pommer, Ernst-Heinrich, Dr.**
**Berliner Platz 7**
**D-6703 Limburgerhof (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Ammoniumsalze von Polysäuren, insbesondere Salze, cyclische Amine, Fungizide, die diese Substanzen enthalten, und Verfahren zu ihrer Herstellung und zur Bekämpfung von Pilzen mit Hilfe dieser Verbindungen.

Es ist bekannt, cyclische Amine und ihre Salze mit monomeren Säuren als Fungizide zu verwenden (DE-11 64 152, DE-26 56 757, DE-27 52 096). Die fungizide Aktivität, Wirkungsdauer und Pflanzenverträglichkeit dieser Substanzen ist in manchen Fällen nicht ausreichend.

Es ist ferner bekannt, Salze fungizider Morpholinderivate mit Tensiden zur Bekämpfung von Pilzen zu verwenden (DE-A-2 461 513). Hierdurch werden die erfindungsgemäßen Salze und ihre Verwendung als Fungizide nicht nahegelegt, da diese sich von den bekannten Salzen sowohl im Morpholin- als auch im Säurerest unterscheiden.

Es wurde nun gefunden, daß Ammoniumsalze von Polysäuren der allgemeinen Formel I

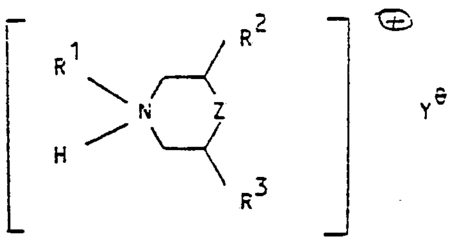

(I)

in der $R^1$ den 3-p-tert.-Butylphenyl-2-methyl-propylrest, $R^2$ und $R^3$ Wasserstoff, Hydroxy-, $—CH_2OH$ oder einen $C_1$-$C_3$-Alkylrest, Z = $CH_2$, O, S, CO, $(CH_2)_2$ und Y ein Anion einer copolymeren Säure auf der Basis von (Meth)acrylsäure, Maleinsäure (anhydrid), Vinylsulfonsäure, Styrolsulfonsäure, (Meth-)allylsulfonsäure, Acrylamido-2-methylpropansulfonsäure, Vinylphosphonsäure und ihrer Salze und ihren Gemischen und eines handelsüblichen Vinyl- oder Acrylmonomeren bedeutet, deutlich stärker wirksam sind als die bisher bekannten cyclischen Amine und ihre Salze mit monomeren Säuren. Die neuen Salze sind wasserlöslich oder in Wasser dispergierbar.

Beispiele für $R^2$ und $R^3$ sind Wasserstoff, Methyl, Ethyl, Propyl, OH, $CH_2OH$.

Handelsübliche Vinyl- und Acrylmonomere sind z. B. Styrol, Vinylacetat, Vinylpropionat, Vinylchlorid, (Meth)acrolein, (Meth)acrylnitril, (Meth)acrylamid und seine Derivate sowie Malon-, Fumarsäureester und (Meth)acrylsäureester, wobei unter den copolymerisierbaren Estern diejenigen vorgezogen werden, die in der Alkohol-Komponente noch freie OH-Gruppen oder Polyethergruppen enthalten, z. B. Hydroxalkylester mit 2 bis 8 C-Atomen im Alkylrest der (Meth)acrylsäure oder Verbindungen der allgemeinen Formel II

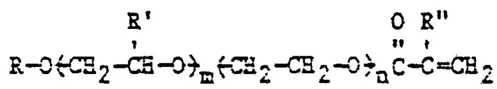

(II)

worin

R = Wasserstoff, $C_1$-$C_{20}$-Alkyl oder $C_1$-$C_{18}$-Alkylphenyl

R′ = Methyl oder Ethyl

R″ = Wasserstoff oder Methyl und

n und m Zahlen zwischen 0 und 100 bedeuten.

Vor allem die letztgenannten monomeren Bausteine tragen erheblich zur Wasserlöslichkeit oder Wasserdispergierbarkeit der neuen Salze bei.

Bevorzugt werden Copolymerisate einer Säure, insbesondere Vinylphosphonsäure oder Acrylamido-2-methylpropansulfonsäure mit einem Acrylester, der in den Alkoholrest des Esters einen höheren Alkylrest, z. B. $C_{16}$-$C_{20}$-Alkyl, insbesondere $C_{18}$-Alkyl, und 60 bis 100, insbesondere 80, Ethylenoxidgruppen ($CH_2—CH_2—O$) enthält. Das Mischungsverhältnis der Monomeren (Säure : Ester) kann in weiten Grenzen schwanken und z. B. 20 : 80 bis 80 : 20 Gew.-Teile, insbesondere 33 : 67 bis 67 : 33 sein.

Die Wirkstoffe der allgemeinen Formel I, die in dem Rest $R^1$ ein asymmetrisches Kohlenstoffatom enthalten, können in Form verschiedener Enantiomere bzw. Diastereomere vorliegen. Die reinen Enantiomere und Diasteriomere sowie ihrer Gemische werden von der Erfindung umfaßt.

Bevorzugt werden Salze mit N-(3-p-tert.-Butylphenyl-2-methyl-propyl)-2,6-cis-dimethylmorpholin.

Die Herstellung der copolymeren Säuren kann nach den üblichen Verfahren der radikalischen Polymerisation in Substanz, Lösung, Emulsion oder Suspension unter Einsatz üblicher Initiatoren (z. B. anorganischen oder organischen Peroxiden, labilen C—C- oder Azo-Verbindungen) und Hilfsmittel wie Emulgatoren, Schutzkolloiden sowie Reglern z. B. labilen Chlor- oder Bromverbindungen, Alkoholen, Mercaptanen erfolgen. Solche Regler werden eingesetzt, um den Grad der Polymerisation zu erniedrigen. Das ist z. B. bei der Verwendung der neuen Salze in Form ihrer Lösungen wichtig, um die Viskosität der

Lösungen im Interesse einer guten Handhabbarkeit möglichst niedrig zu halten.

Als Maß für den Polymerisationsgrad der copolymeren Säuren kann der K-Wert nach Fikentscher dienen [Cellulose Chemie, Band 13 (1932), S. 48 bis 64 und Seite 71 bis 74]. Es wird in 1 %igen (Gew.%) wäßrigen Lösungen bei 25 °C gemessen ; dabei bedeutet $K = 10^3$ k. Die so bestimmten K-Werte der Säuren liegen beispielsweise im Bereich von 10 bis 50, vorzugsweise von 10 bis 30.

Die Umsetzung der basischen Wirkstoffe mit den Polysäuren zu den neuen Salzen erfolgt beispielsweise nach Beendigung der Polymerisation durch einfaches Mischen der fertigen Komponenten, wobei dann auch noch andere Formulierungshilfsmittel wie Emulgatoren, Netzmittel, Trägermaterialien, Stabilisatoren, Lösungsmittel zugesetzt werden können. In bestimmten Fällen kann die Salzbildung vorteilhaft auch bereits vor der Polymerisation zwischen der Base und den sauren Monomeren erfolgen. Voraussetzung dafür ist, daß das entstehende monomere Salz copolymerisierbar, der Wirkstoff unter Polymerisationsbedingungen stabil und frei von Verunreinigungen ist, die die nachfolgende Copolymerisation stören könnten.

Die neuen Salze ergeben beim Lösen in Wasser in den meisten Fällen bereits ohne Verwendung weiterer Formulierungshilfsstoffe stabile Spritzbrühen, die auf den pflanzlichen Substraten zu gut haftenden wirkstoffhaltigen polymeren Überzügen mit guter Pflanzenverträglichkeit antrocknen. Der pH-Wert der wäßrigen Lösungen der Salze beträgt 6 bis 8. Die Fungizide können neben den neuen Salzen auch noch die freien Amine enthalten, von denen sich die Salze ableiten.

Die folgenden Beispiele sollen die Herstellung der neuen Salze weiter erläutern.

### Beispiel 1

In einem Polymerisationsreaktor mit Stickstoffeinleitung, Thermometer, Rührer und Rückflußkühler werden eingetragen 70 Teile (Gew.-Teile) Wasser, 70 Teile Acrylamido-2-methylpropansulfonsäure, 140 Teile Isopropanol, 140 Teile eines Acrylesters der allgemeinen Formel II bei dem $R = C_{18}$-Alkyl, $m = 0$ und $n = 80$ ist, 4,2 Teile Hydroxylammoniumsulfat und 7 Teile Azobisisobuttersäurenitril und auf 80 °C mit Hilfe eines Wasserbades erwärmt. Man hält die Mischung unter einem leichten Stickstoffstrom 3 Stunden lang bei dieser Temperatur und kühlt dann ab. Man erhält eine klare Polymerlösung von einem Feststoffgehalt von 50 % (Gew.%) und einem K-Wert von 26.

In einen Rührkolben mit Wasserbad gibt man zu 100 Teilen der oben hergestellten Polymerlösung unter Rühren bei 40 °C langsam 29 Teile des Wirkstoffs N-(3-p-tert. Butylphenyl-2-methyl-propyl)-2,6-cis-dimethylmorpholin (Fenpropimorph) innerhalb von etwa 1 Stunde zu und rührt noch etwa 1 Stunde lang nach. Nach dem Abkühlen erhält man eine klare Lösung des Salzes mit einem Wirkstoffgehalt von 22,5 %, die bei Verdünnung mit Wasser eine stabile Spritzbrühe ergibt.

### Beispiel 2

In die Apparatur von Beispiel 1 werden eingetragen : 105 Teile Wasser, 70 Teile Vinylphosphonsäure, 105 Teile Isopropanol und 140 Teile des Acrylesters wie in Beispiel 1 beschrieben sowie 7 Teile Azobisisobuttersäurenitril. Man arbeitet analog wie in Beispiel 1 und erhält eine Polysäure-Lösung mit dem Feststoffgehalt von 50,4 % und einem K-Wert von 21.

Wie in Beispiel 1 beschrieben, gibt man zu 100 Teilen der oben hergestellten Polysäure-Lösung 45 Teile Wirkstoff Fenpropimorph. Man erhält eine Polysalz-Lösung mit einem Wirkstoffgehalt von 31 %, die bei Verdünnung mit Wasser eine stabile Spritzbrühe ergibt.

### Beispiel 3

In der Apparatur von Beispiel 1 werden eingetragen : 35 Teile Wasser, 35 Teile Isopropanol, 70 Teile Vinylphosphonsäure und 140 Teile des Acrylesters wie in Beispiel 1, sowie 7 Teile Azobisisobuttersäurenitril. Nach dem Verfahren von Beispiel 1 erhält man eine Polysäure-Lösung mit dem Feststoffgehalt von 73 % und einem K-Wert von 20.

Wie in Beispiel 1 gibt man zu 100 Teilen der oben hergestellten Polysäure-Lösung, 68 Teile Wirkstoff Fenpropimorph und erhält eine Lösung mit einem Wirkstoffgehalt von 40 %.

### Beispiel 4

In der Apparatur von Beispiel 1 werden 35 Teile Wasser, 35 Teile Isopropanol, 140 Teile Vinylphosphonsäure, 70 Teile des Acrylesters wie in Beispiel 1 und 7 Teile Azobisisobuttersäurenitril zu einer Polysäure-Lösung mit einem Feststoffgehalt von 74 % und einem K-Wert von 16 umgesetzt.

Wie in Beispiel 1 setzt man 100 Teile der oben hergestellten Lösung mit 67 Teilen Wirkstoff Fenpropimorph zur Polysalzlösung um, die 40 % Wirkstoff enthält und bei Verdünnung mit Wasser eine stabile Spritzbrühe liefert.

### Beispiel 5

Wie in Beispiel 1 beschrieben, gibt man zu 100 Teilen der in Beispiel 1 hergestellten Polymerlösung unter Rühren bei 40 °C langsam ein Gemisch von je 14,5 Teilen Fenpropimorph und 14,5 Teilen Tridemorph (N-iso-Tridecyl-2,6-dimethylmorpholin) innerhalb von etwa 1 Stunde zu und rührt noch etwa 1 Stunde lang nach. Nach dem Abkühlen erhält man eine klare Lösung mit einem Wirkstoffgehalt von ca. 22,5 Gew.%, die beim Verdünnen mit Wasser eine stabile Spritzbrühe ergibt.

Beispiel 6

Man verfährt wie in Beispiel 5, verwendet jedoch eine 1 : 1 Mischung der Wirkstoffe Fenpropimorph und Dodemorph (N-Cyclododecyl-2,6-dimethylmorpholin). Das so erhaltene Präparat ergibt gleichfalls beim Verdünnen mit Wasser eine stabile Spritzbrühe.

Die neuen Salze und die entsprechenden Fungizide sind insbesondere geeignet zur Bekämpfung von Pflanzenkrankheiten, z. B. Erysiphe graminis (echter Mehltau) an Getreide, Erysiphe cichoriacearum (echter Mehltau) an Kürbisgewächsen, Podosphaera leucotricha an Äpfeln, Uncinula necator an Rebe, Erysiphe polygoni an Bohnen, Sphaerotheca pannosa an Rosen, Microsphaera querci an Eichen, Botrytis cinerea and Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam ; sie werden sowohl über die Wurzeln als auch über die Blätter aufgenommen und im Pflanzengewebe transportiert.

Bei der Anwendung des neuen Wirkstoffs zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten kann der Wirkstoff auch in Verbindung mit Kunststoffdispersionen 0,25 %ig bis 5 %ig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90 %.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise :

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino-)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-Furyl-(2)-benzimidazol

2-Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formulamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken ; sie sollten in der Regel möglichst eine feine Verteilung der neuen Wirkstoffe gewährleisten. Die Lösung oder Dispersion in Wasser wird bevorzugt.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon,

Wasser usw. in Betracht. Bevorzugt wird die Lösung in Wasser.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkyl-arylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalin-derivaten mit Formaldehyd, Kondensationsprodukte des Naphthalins bzw. der Naphthalinsulfonsäuren mit Phenol und Formaldehyd, Polyoxyethylen-octyl-phenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Phospolipide Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Der folgende Versuch erläutert die fungizide Wirkung der neuen Verbindungen.

Versuch 1

Winterweizen der Sorte « Caribo » wurde im Herbst auf Freiland-Kleinparzellen von 1,5 $m^2$ Größe ausgesät. Im folgenden April war die Versuchsfläche durch Spontaninfektion mit Weizenmehltau (Erysiphe graminis f.sp. tritici) so stark befallen, daß der Befall mit der Note 4 im unten angegebenen Bewertungsschema der Biologischen Bundesanstalt Braunschweig beschrieben werden konnte. Zu diesem Zeitpunkt wurde eine Behandlung vorgenommen. Mit einem mobilen Spritzrechen wurden pro Parzelle 250 ml wäßrige Spritzbrühe ausgebracht. Pro Konzentration wurde mit 2 Wiederholungen gearbeitet. Im Abstand von 10 Tagen wurden Auswertungen vorgenommen. Die angegebenen Werte sind Durchschnittswerte von drei Einzelwerten.

Bewertungsschema (nach BBA)
1 ohne Befall
2 bis 2,5 % Befall
3 2,5-5 % Befall
4 5-10 % Befall
5 10-15 % Befall
6 15-25 % Befall
7 25-35 % Befall
8 35-67,5 % Befall
9 67,5-100 % Befall

Das Ergebnis des Versuches zeigt, daß die neuen Salze gemäß Beispiel 3 und 4 bei der Anwendung als 0,075 bis 0,1125 %ige (berechnet als Fenpropimorph) Spritzbrühen bei der Prüfung nach 10, 20, 30 und 40 Tagen eine bessere fungizide Wirkung zeigen (1,0-2,7) als Fenpropimorph in Form seiner handelsüblichen Formulierung (1,7-4,3).

**Patentansprüche**

1. Ammoniumsalze von Polysäuren der allgemeinen Formel I

(Siehe Formel Seite 7 f.)

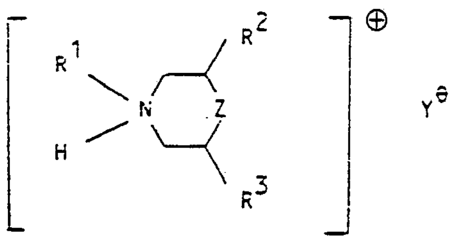

(I)

in der $R^1$ den 3-p-tert.-Butylphenyl-2-methyl-propylrest, $R^2$ und $R^3$ Wasserstoff, Hydroxy-, —$CH_2OH$ oder einen $C_1$-$C_3$-Alkylrest, Z = $CH_2$, O, S, CO, $(CH_2)_2$ und Y ein Anion einer copolymeren Säure auf der Basis von (Meth)acrylsäure, Maleinsäure(anhydrid), Vinylsulfonsäure, Styrolsulfonsäure, (Meth-)allylsulfonsäure, Acrylamido-2-methylpropansulfonsäure, Vinylphosphonsäure und ihrer Salze und ihren Gemischen und eines handelsüblichen Vinyl- oder Acrylmonomeren bedeuten.

2. Fungizides Mittel, enthaltend einen festen oder flüssigen Trägerstoff und ein Salz der allgemeinen Formel I

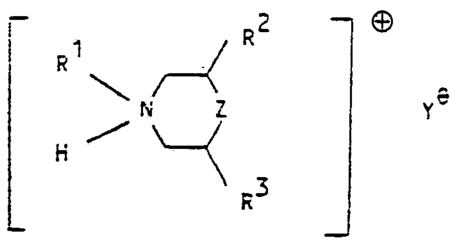

(I)

in der $R^1$ den 3-p-tert.-Butylphenyl-2-methyl-propylrest, $R^2$ und $R^3$ Wasserstoff, Hydroxy-, —$CH_2OH$ oder einen $C_1$-$C_3$-Alkylrest, Z = $CH_2$, O, S, CO, $(CH_2)_2$ und Y ein Anion einer copolymeren Säure auf der Basis von (meth)acrylsäure, Maleinsäure(anhydrid), Vinylsulfonsäure, Styrolsulfonsäure, (Meth-)allylsulfonsäure, Acrylamido-2-methylpropansulfonsäure, Vinylphosphonsäure und ihrer Salze und ihren Gemischen und eines handelsüblichen Vinyl- oder Acrylmonomeren bedeuten.

3. Verfahren zur Bekämpfung von Pilzen, dadurch gekennzeichnet, daß man eine fungizid wirksame Menge einer Verbindung der allgemeinen Formel I

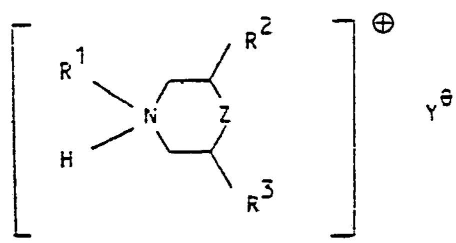

(I)

in der $R^1$ den 3-p-tert.-Butylphenyl-2-methyl-propylrest, $R_2$ und $R_3$ Wasserstoff, Hydroxy-, —$CH_2OH$ oder einen $C_1$-$C_3$-Alkylrest, Z = $CH_2$, O, S, CO, $(CH_2)_2$ und Y ein Anion einer copolymeren Säure auf der Basis von (Meth)acrylsäure, Maleinsäure(anhydrid), Vinylsulfonsäure, Styrolsulfonsäure, (Meth)allylsulfonsäure, Acrylamido-2-methylpropansulfonsäure, Vinylphosphonsäure und ihrer Salze und ihren Gemischen und eines handelsüblichen Vinyl- oder Acrylmonomeren bedeuten, auf Pilze oder durch Pilzbefall bedrohte Materialien, Flächen, Pflanzen oder Saatgüter einwirken läßt.

4. Ammoniumsalz gemäß Anspruch 1, enthaltend ein Kation des Amins N-(3-p-tert.-Butylphenyl-2-methyl-propyl)-2,6-cis-dimethylmorpholin.

**Claims**

1. An ammonium salt of a polymeric acid of the general formula I

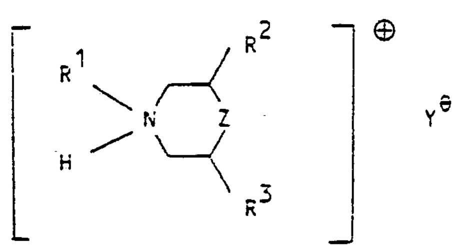

(I)

where $R^1$ is 3-p-tert-butylphenyl-2-methylpropyl, $R^2$ and $R^3$ are each hydrogen, hydroxyl, —$CH_2OH$ or $C_1$-$C_3$-alkyl, Z is $CH_2$, O, S, CO or $(CH_2)_2$ and Y is an anion of a copolymeric acid based on (meth)acrylic acid, maleic acid or anhydride, vinylsulfonic acid, styrenesulfonic acid, (meth)-allylsulfonic acid, acrylamido-2-methylpropanesulfonic acid or vinylphosphonic acid or their salts or mixtures and a commercial vinyl or acrylyl monomer.

2. A fungicide containing a solid or liquid carrier and a salt of the general formula I

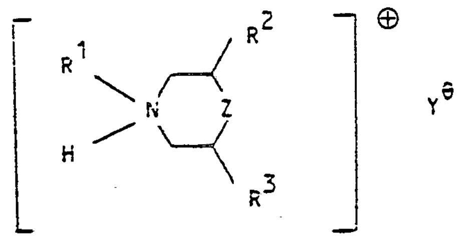

(I)

where $R^1$ is 3-p-tert-butylphenyl-2-methylpropyl, $R^2$ and $R^3$ are each hydrogen, hydroxyl, —$CH_2OH$ or $C_1$-$C_3$-alkyl, Z is $CH_2$, O, S, CO or $(CH_2)_2$ and Y is an anion of a copolymeric acid based on (meth)acrylic acid, maleic acid or anhydride, vinylsulfonic acid, styrenesulfonic acid, (meth)allylsulfonic acid, acrylamido-2-methylpropanesulfonic acid or vinylphosphonic acid or their salts or mixtures and a commercial vinyl or acrylyl monomer.

3. A method for controlling fungi, wherein the fungicidally effective amount of a compound of the general formula I

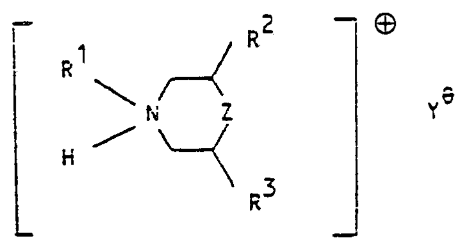

(I)

where $R^1$ is 3-p-tert-butylphenyl-2-methylpropyl, $R^2$ and $R^3$ are each hydrogen, hydroxyl, —$CH_2OH$ or $C_1$-$C_3$-alkyl, Z is $CH_2$, o, S, CO or $(CH_2)_2$ and Y is an anion of a copolymeric acid based on (meth)acrylic acid, maleic acid or anhydride, vinylsulfonic acid, styrenesulfonic acid, (meth)-allylsulfonic acid, acrylamido-2-methylpropanesulfonic acid or vinylphosphonic acid or their salts or mixtures and a commercial vinyl or acrylyl monomer, is allowed to act on fungi or materials, areas, plants or seed threatened by fungal attack.

4. An ammonium salt as claimed in claim 1, containing a cation of the amine N-(3-p-tert-butylphenyl-2-methylpropyl)-2,6-cis-dimethylmorpholine.

**Revendications**

1. Sels ammoniacaux de polyacides de formule générale I

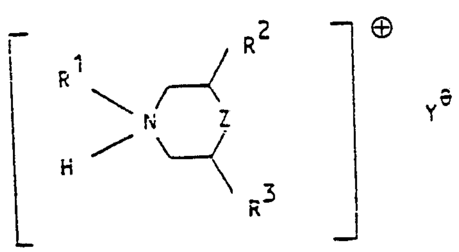

(I)

dans laquelle $R^1$ représente le reste 3-p-tert-butylphényl-2-méthyl-propyle,
$R^2$ et $R^3$ hydrogène, hydroxy-, —$CH_2OH$ ou un reste alkyle en $C_1$-$C_3$,
Z = $CH_2$, O, S, CO, $(CH_2)_2$ et
Y un anion d'un acide copolymère à base d'acide (méth)acrylique, (anhydride)acide maléique, acide vinylsulfonique, acide styrènesulfonique, acide (méth)allylsulfonique, acide acrylamido-2-méthylpropanesulfonique, acide vinylphosphonique et leurs sels et leurs mélanges, et d'un monomère vinylique ou acrylique du commerce.

2. Agent fongicide, contenant un support solide ou liquide et un sel de formule générale I

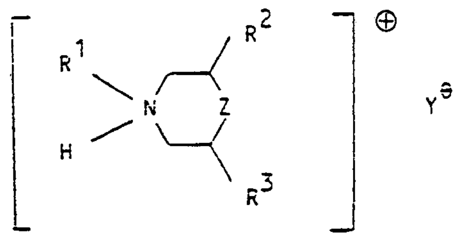

(I)

dans laquelle $R_1$ représente le reste 3-p-tert-butylphényl-2-méthyl-propyle,
$R^2$ et $R^3$ hydrogène, hydroxy-, —$CH_2OH$ ou un reste alkyle en $C_1$-$C_3$,
$Z = CH_2$, O, S, CO, $(CH_2)_2$ et
Y un anion d'un acide copolymère à base d'acide (méth)acrylique, (anhydride)acide maléique, acide vinylsulfonique, acide styrènesulfonique, acide (méth)allylsulfonique, acide acrylamido-2-méthylpropanesulfonique, acide vinylphosphonique et leurs sels et leurs mélanges, et d'un monomère vinylique ou acrylique du commerce.

3. Procédé de lutte contre les champignons, caractérisé par le fait que l'on fait agir sur les champignons ou les matières, surfaces, plantes ou semences menacées par des champignons, une quantité efficace, du point de vue fongicide, d'un composé de formule générale I

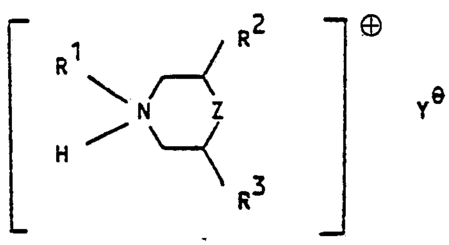

(I)

dans laquelle $R^1$ représente le reste 3-p-tert-butylphényl-2-méthyl-propyle,
$R^2$ et $R^3$ hydrogène, hydroxy-, —$CH_2OH$ ou un reste alkyle en $C_1$-$C_3$,
$Z = CH_2$, O, S, CO, $(CH_2)_2$ et
Y un anion d'un acide copolymère à base d'acide (méth)acrylique, (anhydride)acide maléique, acide vinylsulfonique, acide styrènesulfonique, acide (méth)allylsulfonique, acide acrylamido-2-méthylpropanesulfonique, acide vinylphosphonique et leurs sels et leurs mélanges, et d'un monomère vinylique ou acrylique du commerce.

4. Sel ammoniacal selon la revendication 1, contenant un cation de l'amine N-(3-p-tert-butylphényl-2-méthyl-propyl)-2,6-cis-diméthylmorpholine.